# EUROPEAN PATENT SPECIFICATION

(11) **EP 3 714 790 B1**
(45) Date of publication and mention of the grant of the patent: **09.02.2022**
(21) Application number: 19177989.1
(22) Date of filing: 03.06.2019
(51) Int. Cl.: A61B 5/15, A61B 5/154

(54) **PEN-TYPE BACKFLOW-PROOF RETRACTABLE FLOW VISIBLE BLOOD COLLECTION NEEDLE**
STIFTARTIGE, RÜCKFLUSSSICHERE, AUSZIEHBARE BLUTENTNAHMENADEL MIT SICHTBAREM FLUSS
AIGUILLE DE PRÉLÈVEMENT SANGUIN À FLUX VISIBLE, RÉTRACTABLE, ANTIREFOULEMENT ET DE TYPE STYLO

(30) Priority: 28.03.2019 CN 201920458071 U
(43) Date of publication of application: 30.09.2020
(73) Proprietor: Liu, Zhenkuan, Heze City, Shandong Province 274200 (CN)
(72) Inventor: Liu, Zhenkuan, Heze City, Shandong Province 274200 (CN)
(74) Representative: Jannig & Repkow Patentanwälte PartG mbB

(56) References cited:
- EP-A1- 0 329 038
- US-A- 5 423 758
- US-A- 5 695 475
- US-A- 5 769 826
- US-A1- 2011 178 427

## Description

### TECHNICAL FIELD

The present invention relates to the technical field of medical equipment, especially relates to a pen-type backflow-proof retractable flow visible blood collection needle.

### BACKGROUND TECHNOLOGY

Currently, blood collection needles are used to collect samples of human blood; however, with the currently available needle holders for holding the blood collection needles, it is not possible to insulate and protect the exposing needle tips, which is liable to hurt the staff members after completing blood collection. Meanwhile, as the one-way valve seat is opaque, it is not possible for the medical staff to determine whether the venous needle has been correctly injected into the blood vessel. Therefore, a pen-type backflow-proof retractable flow visible blood collection needle is provided.

### SUMMARY OF THE INVENTION

The object of the present invention is to address the problems existing in the prior art, and to provide a pen-type backflow-proof retractable flow visible blood collection needle.

In order to achieve the above object, the invention adopts the following technical solution:
Pen-type backflow-proof retractable flow visible blood collection needle, comprises a blood collection needle, wherein the blood collection needle comprises an injection end protection case and butting end protection case, the bottom of the butting end protection case is open, inside which is provided a one-way valve seat, on top of the one-way valve seat is provided a backflow proof one-way valve, and on top of the backflow proof one-way valve is provided a blood collection needle; at the inner side of the top end of the injection end protection case is fixed a spring; to the lower end of the one-way valve seat is provided the blood collection needle seat, four sides of the blood collection needle seat are provided with fixing blocks, and on the side wall of the butting end protection case are provided four fixing cakes, four fixing blocks correspond with four fixing cakes; an opening is provided from bottom to top of the blood collection needle seat, the said opening comprises a threaded hole, a first rectangular hole and a second rectangular hole, in the threaded hole is connected a threaded rod; to the bottom of the one-way valve seat is connected a connection pipe, which goes through the threaded hole, and extends to the first rectangular hole and is connected with a flexible socket pipe; to the bottom of the flexible socket pipe is connected the blood collection tube, top of which reaches and contacts the inner side of the top of the second rectangular hole; on the blood collection device is provided an operation hole; on top of the blood collection device is provided a protection cover; inside the butting end protection case is provided a ring-shaped spacer.

Preferably, the top end of the injection end protection case is provided with a first circular opening, of which radius is larger than that of the tip of the blood collection needle.

Preferably, all of the blood collection device, the one-way valve seat and the backflow proof one-way valve are made of transparent polycarbonate.

Compared with the prior art, the beneficial effects of the invention are:
With the coordination of the blood collection device, injection end protection case, butting end protection case, one-way valve seat, backflow proof one-way valve, tip of the blood collection needle, spring, blood collection needle seat, opening, threaded rod, collection pipe, flexible socket pipe, blood collection tube, fixing blocks, fixing cakes, operation hole, protection cover and spacer, the present blood collection device may work in such a way: excise power and push the blood collection tube, which will move the blood collection needle seat upwards together with the fixing blocks; in the meantime, the blood collection blood seat moves the backflow proof one-way valve upwards with the one-way valve seat; the backflow proof one-way valve in turn drives the tip of the blood collection needle upwards and compresses the spring; when the tip of the blood collection needle reaches out from the first circular opening, turn the blood collection tube, which in turn rotates the fixing blocks with the blood collection needle seat, and when the fixing blocks rotate to the appropriate position, release the blood collection tube, and restoration of the compressed spring will drive the blood collection needle seat downwards, which brings the fixing blocks downwards against corresponding fixing cakes; when collecting blood samples, it is possible to perceive visually whether blood flows into the blood collection tube, and knows whether the venous injection needle is correctly injected into the blood tube; after blood collection, turn the blood collection needle seat from the operation hole to separate the fixing blocks from the corresponding fixing cakes, simultaneously, compressed spring release will withdraw the tip of the blood collection needle back into the blood collection device, preventing it from being exposed and hurting any working staff.

The present invention is of simple structure, and easy operation. With the present invention, it is convenient to withdraw the tip of the blood collection needle to present hurting anyone and perceive visually whether blood flows to the blood collection tube, which is user friendly and contributory.

### BRIEF INTRODUCTION OF THE DRAWINGS

Figure 1 is a schematic structural view of the pen-type backflow-proof retractable flow visible blood collection needle according to the present invention;
Figure 2 is a schematic structural view of the portion A of the pen-type backflow-proof retractable flow visible blood collection needle according to the present invention;
Figure 3 is a schematic structural view of the appearance of the pen-type backflow-proof retractable flow visible blood collection needle according to the present invention.

The drawings includes following integers:
1- Blood collection device; 2- injection end protection case; 3- butting end protection case; 4- one-way valve seat; 5- backflow proof one-way valve; 6- blood collection needle; 7- spring; 8- blood collection needle seat; 9- opening; 10- threaded rod; 11-connecting pie; 12- flexible socket pipe; 13- blood collection tube; 14- fixing block; 15- fixing cake; 16- operation hole; 17- protection cover; 18- spacer.

### DETAILED DESCRIPTION

The technical solutions in the embodiments of the present invention will be clearly and completely described below in conjunction with the accompanying drawings in the embodiments of the present invention. It is apparent that the described embodiments are only a part of the embodiments of the present invention, and are not all of the embodiments.

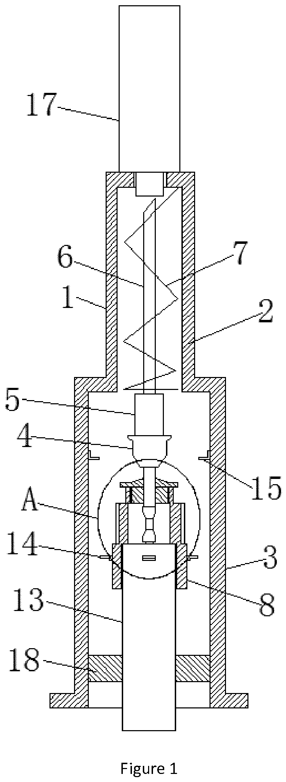

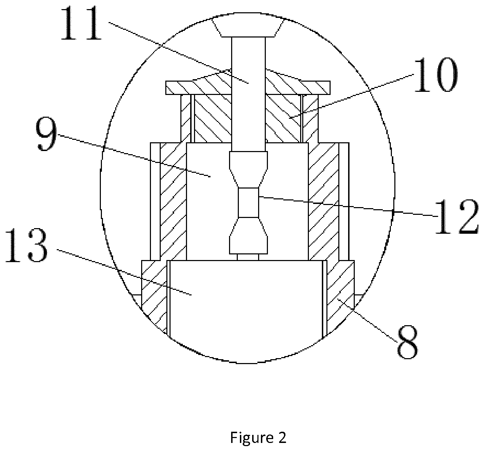

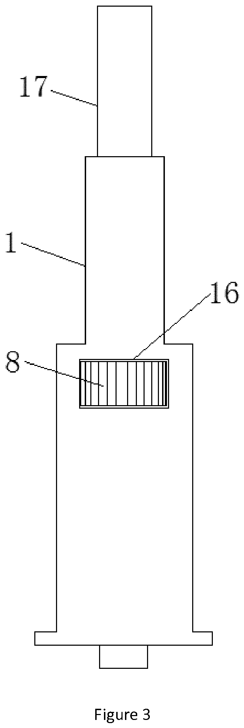

Referring to figures 1-3, a pen-type backflow-proof retractable flow visible blood collection needle, comprises a blood collection needle 1, wherein the blood collection needle 1 comprises an injection end protection case 2 and butting end protection case 3, the bottom of the butting end protection case is open, inside which is provided a one-way valve seat 4, on top of the one-way valve seat 4 is provided a backflow proof one-way valve 5, and on top of the backflow proof one-way valve 5 is provided a blood collection needle 6; at the inner side of the top end of the injection end protection case 2 is fixed a spring 7; to the lower end of the one-way valve seat 4 is provided the blood collection needle seat 8, four sides of the blood collection needle seat 8 are provided with fixing blocks 14, and on the side wall of the butting end protection case 3 are provided four fixing cakes 15, four fixing blocks 14 correspond with four fixing cakes 15; an opening 9 is provided from bottom to top of the blood collection needle seat 8, the said opening 9 comprises a threaded hole, a first rectangular hole and a second rectangular hole, in the threaded hole is connected a threaded rod 10; to the bottom of the one-way valve seat 4 is connected a connection pipe 11, which goes through the threaded hole, and extends to the first rectangular hole and is connected with a flexible socket pipe 12; to the bottom of the flexible socket pipe 12 is connected the blood collection tube 13 made from transparent material, and the blood collection tube 13 is configured to be of negative pressure; top of the blood collection tube 13 reaches and contacts the inner side of the top of the second rectangular hole; on the blood collection device 1 is provided an operation hole 16; on top of the blood collection device 1 is provided a protection cover 17; inside the butting end protection case 3 is provided a ring-shaped spacer 18; the top end of the injection end protection case 2 is provided with a first circular opening, of which radius is larger than that of the tip of the blood collection needle 6; all of the blood collection device 1, the one-way valve seat 4 and the backflow proof one-way valve 5 are made of transparent polycarbonate. The present invention is of simple structure, and easy operation. With the present invention, it is convenient to withdraw the tip of the blood collection needle to present hurting anyone and perceive visually whether blood flows to the blood collection tube, which is user friendly and contributory.

Working principle of the present invention: to use the present blood collection device 1, excise power and push the blood collection tube 13, which will move the blood collection needle seat 8 upwards together with the fixing blocks 14; in the meantime, the blood collection blood seat 8 moves the backflow proof one-way valve 5 upwards with the one-way valve seat 4; the backflow proof one-way valve 5 in turn drives the tip of the blood collection needle 6 upwards and compresses the spring 7; when the tip of the blood collection needle 6 reaches out from the first circular opening, turn the blood collection tube 13, which in turn rotates the fixing blocks 14 with the blood collection needle seat 8, and when the fixing blocks 14 rotate to the appropriate position, release the blood collection tube 13, and restoration of the compressed spring 7 will drive the blood collection needle seat 8 downwards, which brings the fixing blocks 14 downwards against corresponding fixing cakes 15; when collecting blood samples, it is possible to perceive visually whether blood flows into the blood collection tube 13, and knows whether the venous injection needle is correctly injected into the blood tube; after blood collection, turn the blood collection needle seat 8 from the operation hole 16 to separate the fixing blocks 14 from the corresponding fixing cakes 15, simultaneously, compressed spring 7 release will withdraw the tip of the blood collection needle 6 back into the blood collection device 1, preventing it from being exposed and hurting any working staff.

The above embodiment is only a preferred embodiment of the present invention, but the scope of protection of the present invention is not limited thereto.

## Claims

1. Pen-type backflow-proof retractable flow visible blood collection needle, comprises a blood collection needle (1), wherein the blood collection needle (1) comprises an injection end protection case (2) and butting end protection case (3), the bottom of the butting end protection case is open, inside which is provided a one-way valve seat (4), on top of the one-way valve seat (4) is provided a backflow proof one-way valve (5), and on top of the backflow proof one-way valve (5) is provided a blood collection needle (6); at the inner side of the top end of the injection end protection case (2) is fixed a spring (7); to the lower end of the one-way valve seat (4) is provided the blood collection needle seat (8), four sides of the blood collection needle seat (8) are provided with fixing blocks (14), and on the side wall of the butting end protection case (3) are provided four fixing cakes (15), four fixing blocks (14) correspond with four fixing cakes (15); an opening (9) is provided from bottom to top of the blood collection needle seat (8), the said opening (9) comprises a threaded hole, a first rectangular hole and a second rectangular hole, in the threaded hole is connected a threaded rod (10); to the bottom of the one-way valve seat (4) is connected a connection pipe (11), which goes through the threaded hole, and extends to the first rectangular hole and is connected with a flexible socket pipe (12); to the bottom of the flexible socket pipe (12) is connected the blood collection tube (13) made from transparent material, and the blood collection tube (13) is configured to be of negative pressure; top of the blood collection tube (13) reaches and contacts the inner side of the top of the second rectangular hole; on the blood collection device (1) is provided an operation hole (16); on top of the blood collection device (1) is provided a protection cover (17); inside the butting end protection case (3) is provided a ring-shaped spacer (18)

2. The pen-type backflow-proof retractable flow visible blood collection needle according to claim 1, wherein the top end of the injection end protection case (2) is provided with a first circular opening, of which radius is larger than that of the tip of the blood collection needle (6).

3. The pen-type backflow-proof retractable flow visible blood collection needle according to claim 1, wherein all of the blood collection device (1), the one-way valve seat (4) and the backflow proof one-way valve (5) are made of transparent polycarbonate.

## Patentansprüche

1. Rückflusssichere, zurückziehbare Blutentnahmenadel von Pen-Typ mit sichtbarem Fluss, umfassend eine Blutentnahmenadel (1), wobei die Blutentnahmenadel (1) eine Injektionsende-Schutzhülle (2) und eine Anschlagende-Schutzhülle (3) umfasst, wobei die Unterseite der Anschlagende-Schutzhülle offen ist, wobei in dieser ein Einwegventil-Sitz (4) angeordnet ist, wobei an der Oberseite des Einwegventil-Sitzes (4) ein rückflusssicheren Einwegventil (5) angeordnet ist und an der Oberseite des rückflusssicheren Einwegventils (5) eine Blutentnahmenadel (6) angeordnet ist; wobei an der Innenseite des oberen Endes der Injektionsende-Schutzhülle (2) eine Feder (7) angebracht ist; wobei an dem unteren Ende des Einwegventil-Sitzes (4) der Blutentnahmenadel-Sitz (8) angeordnet ist, an vier Seiten des Blutentnahmenadel-Sitzes (8) Fixierblöcke (14) angeordnet sind und an der Seitenwand der Anschlagende-Schutzhülle (3) vier Fixierplatten (15) angeordnet sind, wobei vier Fixierblöcke (14) vier Fixierplatten (15) entsprechen; wobei eine Öffnung (9) von der Unterseite zu der Oberseite des Blutentnahmenadel-Sitzes (8) angeordnet ist, wobei die Öffnung (9) ein Gewindeloch, ein erstes rechteckiges Loch und ein zweites rechteckiges Loch umfasst, wobei in dem Gewindeloch ein Gewindestab (10) angebracht ist; wobei die Unterseite des Einwegventil-Sitzes (4) mit einem Verbindungsrohr (11) verbunden ist, das durch das Gewindeloch verläuft und bis zu dem ersten rechteckigen Loch verläuft und mit einem flexiblen Muffenrohr (12) verbunden ist; wobei die Unterseite des flexiblen Muffenrohrs (12) mit dem Blutsammeirohr (13) verbunden ist, das aus einem transparenten Material besteht, und das Blutsammelrohr (13) dafür ausgelegt ist, Unterdruck aufzuweisen; wobei die Oberseite des Blutsammelrohrs (13) die Innenseite der Oberseite des zweiten rechteckigen Lochs erreicht und damit in Kontakt steht; wobei an der Blutentnahmevorrichtung (1) ein Betätigungsloch (16) angeordnet ist; wobei an der Oberseite der Blutentnahmevorrichtung (1) eine Schutzabdeckung (17) angeordnet ist; wobei in der Anschlagende-Schutzhülle (3) ein ringförmiger Abstandhalter (18) angeordnet ist.

2. Rückflusssichere, zurückziehbare Blutentnahmenadel von Pen-Typ mit sichtbarem Fluss gemäß Anspruch 1, wobei das obere Ende der Injektionsende-Schutzhülle (2) eine erste kreisförmige Öffnung aufweist, deren Radius größer als jener der Spitze der Blutentnahmenadel (6) ist.

3. Rückflusssichere, zurückziehbare Blutentnahmenadel von Pen-Typ mit sichtbarem Fluss gemäß Anspruch 1, wobei alle von der Blutentnahmevorrichtung (1), dem Einwegventil-Sitz (4) und dem rückflusssicheren Einwegventil (5) aus transparentem Polycarbonat bestehen.

## Revendications

1. Aiguille de prélèvement sanguin à écoulement visible, rétractable, anti-reflux, de type stylo, comprenant une aiguille de prélèvement sanguin (1), dans laquelle l'aiguille de prélèvement sanguin (1) comprend une enveloppe de protection d'extrémité d'injection (2) et une enveloppe de protection d'extrémité d'aboutement (3), le bas de l'enveloppe de protection d'extrémité d'aboutement est ouvert, un siège de soupape de retenue (4) est disposé à l'intérieur de celle-ci, une soupape de retenue anti-reflux (5) est disposée au-dessus du siège de soupape de retenue (4), et une aiguille de prélèvement sanguin (6) est disposée au-dessus de la soupape de retenue anti-reflux (5) ; un ressort (7) est fixé sur le côté intérieur de l'extrémité supérieure de l'enveloppe de protection d'extrémité d'injection (2) ; le siège d'aiguille de prélèvement sanguin (8) est disposé à l'extrémité inférieure du siège de soupape de retenue (4), les quatre côtés du siège d'aiguille de prélèvement sanguin (8) sont pourvus de blocs de fixation (14), et quatre barres de fixation (15) sont disposées sur la paroi latérale de l'enveloppe de protection d'extrémité d'aboutement (3), les quatre blocs de fixation (14) correspondent aux quatre barres de fixation (15) ; une ouverture (9) est ménagée de bas en haut du siège d'aiguille de prélèvement sanguin (8), ladite ouverture (9) comprend un trou taraudé, un premier trou rectangulaire et un deuxième trou rectangulaire, une tige filetée (10) est raccordée dans le trou taraudé ; un tube de raccordement (11) est raccordé au bas du siège de soupape de retenue (4), lequel tube traverse le trou taraudé, et s'étend jusqu'au premier trou rectangulaire et est raccordé à un tube à emboîture souple (12) ; le tube de prélèvement sanguin (13) constitué d'un matériau transparent est raccordé au bas du tube à emboîture souple (12), et le tube de prélèvement sanguin (13) est configuré pour être en dépression ; le dessus du tube de prélèvement sanguin (13) atteint et touche le côté intérieur du dessus du deuxième trou rectangulaire ; un trou de fonctionnement (16) est ménagé sur le dispositif de prélèvement sanguin (1) ; un capuchon de protection (17) est disposé sur le dessus du dispositif de prélèvement sanguin (1) ; une entretoise en forme de bague (18) est disposée à l'intérieur de l'enveloppe de protection d'extrémité d'aboutement (3).

2. Aiguille de prélèvement sanguin à écoulement visible, rétractable, anti-reflux, de type stylo selon la revendication 1, dans laquelle l'extrémité supérieure de l'enveloppe de protection d'extrémité d'injection (2) est pourvue d'une première ouverture circulaire, dont le rayon est plus grand que celui de la pointe de l'aiguille de prélèvement sanguin (6).

3. Aiguille de prélèvement sanguin à écoulement visible, rétractable, anti-reflux, de type stylo selon la revendication 1, dans laquelle le dispositif de prélèvement sanguin (1), le siège de soupape de retenue (4) et la soupape de retenue anti-reflux (5) sont constitués en totalité de polycarbonate transparent.
